Europäisches Patentamt

European Patent Office

Office européen des brevets

(19)

(11) Veröffentlichungsnummer: **0 109 627**
**B1**

(12) **EUROPÄISCHE PATENTSCHRIFT**

(45) Veröffentlichungstag der Patentschrift: **25.05.88**

(51) Int. Cl.⁴: **A 61 H 31/00,** A 61 M 16/00

(21) Anmeldenummer: **83111295.8**

(22) Anmeldetag: **11.11.83**

(54) **Respirator zur Beatmung eines Patienten im Herzrhythmus und zur Unterstützung der Blutzirkulation.**

(30) Priorität: **19.11.82 DE 3242814**

(43) Veröffentlichungstag der Anmeldung:
**30.05.84 Patentblatt 84/22**

(45) Bekanntmachung des Hinweises auf die
Patenterteilung:
**25.05.88 Patentblatt 88/21**

(84) Benannte Vertragsstaaten:
**CH DE FR GB IT LI SE**

(56) Entgegenhaltungen:
**EP-A-0 029 352**
**EP-A-0 057 924**
**FR-A-1 535 612**
**US-A-2 588 192**
**US-A-3 461 860**
**US-A-3 461 861**
**US-A-3 651 801**
**US-A-4 349 015**

(73) Patentinhaber: **Siemens-Elema AB**
**Röntgenvägen 2**
**S-171 95 Solna 1 (SE)**
(84) **SE**

(73) Patentinhaber: **Siemens Aktiengesellschaft**
**Berlin und München**
**Wittelsbacherplatz 2**
**D-8000 München 2 (DE)**
(84) **CH DE FR GB IT LI**

(72) Erfinder: **Olsson, Sven Gunnar**
**Postlada 652**
**S-24017 Södra Sandby (SE)**
Erfinder: **Jonson, Björn, Dr.**
**Nicoloviusväg 11**
**S-22365 Lund (SE)**

Courier Press, Leamington Spa, England.

EP 0 109 627 B1

**Beschreibung**

Die Erfindung betrifft einen Respirator zur Beatmung eines Patienten im Herzrhythmus und zur Unterstützung der Blutzirkulation gemäss dem Oberbegriff des Patentanspruches 1.

Bei konventionellen Respiratorbehandlungen wird den Lungen Atemgas unter Druck zugeführt. Dieser Druck pflanzt sich teilweise auch in die Körperhohlräume im Brustkorb ausserhalb der Lungen fort. Es ist bekannt, dass dieser Druck im wesentlichen die Blutzirkulation negativ beeinflusst. Das beruht hauptsächlich darauf, dass die Blutzufuhr zum Herzen und das Füllen desselben während der Entspannungsphase, der Diastole, behindert wird. Eine ungenügende Füllung der Herzkammern bedeutet aber, dass das Herz während der darauf folgenden Kontaktionsphase, de Systole, kein adäquates Schlagvolumen erzeugen kann. Wenn die Herztätigkeit oder die Blutzirkulation aufgrund von Krankheiten bereits unzureichend sind, können die negativen Einflüsse bei der Respiratorbehandlung schwerwiegende Folgen haben.

Um diese schädlichen Effekte auf die Zirkulation zu vermindern, sind bereits eine Reihe von Vorschlägen gemacht worden. So ist es bekannt, dass eine ausreichende Beatmung mit sehr hohen Atemfrequenzen, z.B. mehreren Atemzügen pro Sekunde, erzielt werden kann. Dabei nimmt das Volumen jedes Atemzuges ab und damit die für die Blutzirkulation störenden Drucksteigerungen. (Proc. Am. Soc. Exp. Biol. 38: 951, 1979: Prospekt AGA Bronchovent, 318.002 Sv, Nov. 78: Klain Jet Ventilator).

Aus der EP—A—57 924 ist es darüberhinaus bekannt, eine hochfrequente Beatmung synchron zu natürlichen Herzaktivitäten vorzunehmen. Da dabei bereits ein niedriges Atemzugvolumen eine ausreichende Beatmung ergibt, kommt nur eine sehr geringe intrathorakale Drucksteigerung zustande. Selbst wenn diese Drucksteigerung mit dem Herzrhythmus synchronisiert würde, so dass sie während der Systole aufträte, würde nur ein geringer positiver Effekt erzielt.

Bei einer Vergrösserung des Atemzugvolumens — um eine effektivere Drucksteigerung zu erzielen — besteht das Risiko, die Lungen durch den höheren Gasdruck, dem sie dann ausgesetzt sind, zu beschädigen. Ausserdem können die Lungen bei derart grossen Atemzugvolumina nicht schnell genug entleert werden.

Weiterhin ist es bekannt, dass durch externe Kompression des Brustkorbes die Herzkammern entleert werden können, die sich bei Zurücknahme dieser Kompression wieder füllen. Dadurch kann eine gewisse Blutzirkulation auch dann aufrechterhalten werden, wenn das Herz nicht selbständig kontrahiert oder in seiner Funktion stark herabgesetzt ist. Aus der US—A—4,349,015 ist ein derartiger Wiederbelegungsapparat bekannt, mit dessen Hilfe bei Herzstillstand der Brustkorb mechanisch stark komprimiert wird und gleichzeitig mit der mechanischen Kompression Atemgas in die Lungen gepumpt wird. Bei der ersten Atemgaszufuhr wird gleichzeitig eine Bauchmanschette aufgepumpt. Eine Synchronisierung mit Herzaktivitäten ist wegen des fehlenden Herzschlages hier jedoch nicht möglich. Die Verletzungsgefahr des Brustkorbes oder innerer Organe durch die starke Kompression wird bei einem solchen Wiederbelebungsapparat bewusst in Kauf genommen.

Der vorliegenden Erfindung liegt die Aufgabe zugrunde, bei einem Respirator der eingangs genannten Art den auf das Herz ausgeübten intrathorakalen Druck einstellbar so weit zu steigern, dass die Kontraktion des Herzens wesentlich vergrössert wird, ohne dass Schäden an den Lungen oder den Brustkorbwänden auftreten. Diese Aufgabe wird erfindungsgemäss durch die kennzeichnenden Merkmale des Anspruches 1 gelöst.

Die Erfindung geht dabei von der Erkenntnis aus, dass die gewünschte Drucksteigerung um das Herz herum durch eine synchrone Beatmung im Herzrhythmus zumindest teilweise dadurch wieder aufgehoben wird, dass sich während der Beatmung der Brustkorb und der Bauch ausdehnen können. Die Erfindung sieht daher vor, dass diese Ausdehnung begrenzt wird, indem eine Kraft auf den Brustkorb und/oder den Bauch ausgeübt wird. Die Lungen müssen sich daher bei der Beatmung teilweise nach innen ausdehnen, so dass die Drucksteigerung um das Herz herum in dem angestrebten Masse erzielt wird. Die Beatmung des Patienten soll dabei im Herzrhythmus erfolgen. Darunter soll verstanden werden, dass pro Herzzyklus ein Atemzug vorliegt oder aber auch, dass die Atemzüge jeweils nur bei jedem zweiten Herzschlag auftreten oder noch seltener, in jedem Fall aber synchron zur Herztätigkeit.

Bei dem erfindungsgemässen Respirators besteht die weitere Anordnung aus einer Reihe von um den Brustkorb und/oder Bauch des Patienten herum angeordneten geschlossenen Kammern aus einem flexiblen Material, die mit einer Flüssigkeit oder einem Gas füllbar sind. Je nach dem Füllgrad kann dabei praktisch die Innenabmessung, d.h. die Grösse des Hohlkörpers, festgelegt werden. Dadurch wird eine gesteigerte Anpassbarkeit des Respirators und insbesondere auchh die Anwendungsmöglichkeiten ein und derselben weiteren Anordnung für verschiedene Patienten ermöglicht. Um den Einfluss auf die Blutzirkulation noch weiter erhöhen zu können, sind Mittel zum Füllen bzw. Entleeren der Kammern vorgesehen. Dadurch kann die auf Brustkorb und/oder Bauch ausgeübte Kraft zeitlich exakt auf die Systole begrenzt und danach durch Entleeren der Kammern schnell aufgehoben werden. Obwohl sich die Lungen dann beispielsweise noch in einem mehr oder weniger gefüllten Zustand befinden, wird kein wesentlicher Druck mehr auf das Herz ausgeübt, da sich der Brustkorb wieder ausdehnen kann.

Um die Beatmung und/oder gleichzeitig die Unterstützung der Blutzirkulation zu optimieren, ist in Weiterbildung der Erfindung vorgesehen, dass der Zeitpunkt und/oder die Dauere der Atemgaszufuhr und/oder der Druck des Atemgases einstellbar sind. Weiterhin können auch der Zeitpunkt, die Dauer und die Grösse der auf Brustkorb und/odere Bauch ausgeübten Kraft einstellbar sein.

Eine Möglichkeit zur weiteren Drucksteigerung besteht darin, dass der Respirator mit einem variablen Totraum versehen ist. Unter Totraum wird dabei quasi ein Puffer in den Atemgasleitungen verstanden, der bei der Ausatmung ein Teilvolumen $CO_2$ speichert und während der folgenden Einatmungsphase wieder an die Lungen abgibt. Im einfachsten Fall kann es sich um eine Verlängerung des Schlauches zwischen der Trachealkanüle und dem Respirator handeln.

Auf diese Weise kann einem Patienten Atemgas mit einem höheren Druck zugeführt werden, so dass die Entleerung des Herzens während der Stystole weiter gesteigert wird und ohne dass der $CO_2$-Gehalt in der Lungen auf ein störendes Niveau absinkt.

Weitere Augestaltungen der Erfindung ergeben sich aus den Unteransprüchen.

Im folgenden wird anhand einer Figur ein Ausführungsbeispiel eines Respirators gemäss der Erfindung näher beschrieben und erläutert.

Die einzige Figur zeigt dabei in einer schematischen Darstellung den an einen Patienten angeschlossenen Respirator.

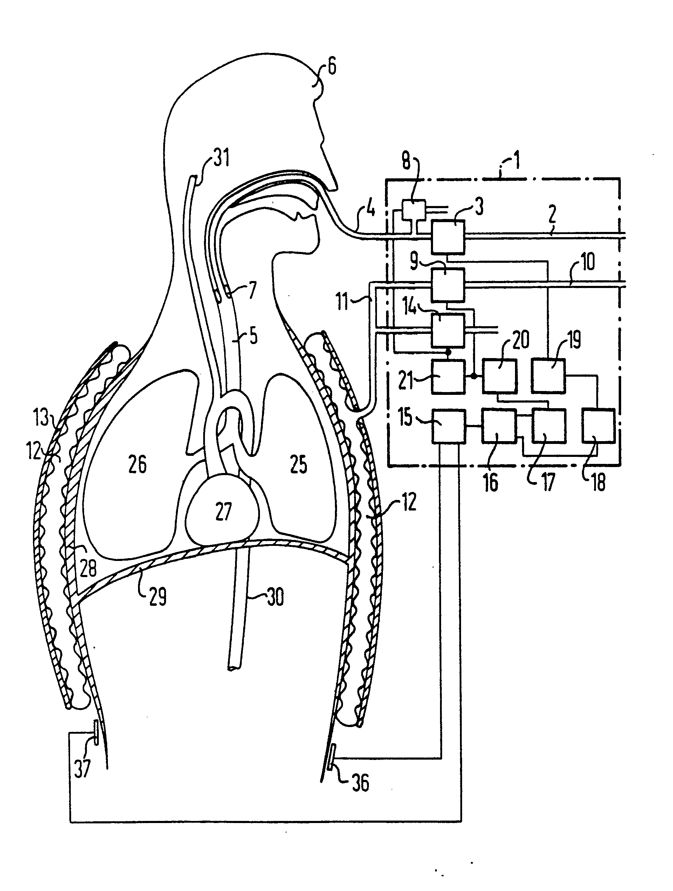

Figur 1 zeigt gestrichelt einen Respirator 1, dem über eine Leitung 2 Atemgas von einer hier nicht dargestellten Gasquelle zugeführt wird. An Stelle einer einzelnen Gasleitung können auch eine Mehrzahl derartiger Leitungen für verschiedene Komponenten des Gases vörgesehen sein. Ueber eine Ventilanordnung 3 sowie eine daran angeschlossene Trachealkanüle 4, die dicht in der Trachea 5 eines Patienten 6 abschliesst, wird das Atemgas dem Patienten zugeführt. Zum Abschliessen der Trachealkanüle 4 in der Trachea kann beispielsweise eine Manschette 7 vorgesehen sein. An die Trachealkanüle 4 ist weiterhin ein Auslassventil 8 angeschlossen. Die Anordnung 3, die eine dosierte Zufuhr von Atemgas von der nicht dargestellten, an die Leitung 2 angeschlossenen Ueberdruckgasquelle zum Patienten 6 erzeugt, kann beispielsweise von der Art sein, wie sie aus der schwedischen Patentanmeldung Nr. 8101488—0, veröffentlicht unter WO—82/03014, bekannt ist.

Ebenso ist es jedoch auch möglich, anstatt einer in der Trachea abschliessenden Trachealkanüle und eines Auslassventiles eine offene Kanüle für die spontane Atmung mit einer dünnen zusätzlichen Kanüle für die Zufuhr von Atemgas unter Druck zu verwenden. Die Entleerung der Lungen geschieht dann direkt über die offene Kanüle. Weiterhin ist es auch möglich, eine dünne Kanüle auf operativem Weg direkt in die Trachea einzusetzen. Die Ausatmung geschieht dann über die natürlichen Atmungsorgane. In den beiden letzten Fällen wird man vorteilhaft eine HFPPV-Beatmungsmethode (High-Frequency Positiv-Pressure Ventilation) verwenden.

Der Respirator 1 enthält weiterhin ein Ventil 9, dem über eine Leitung 10 Gas von einer weiteren, ebenfalls nicht dargestellten Druckgasquelle zugeführt wird. Im einfachsten Fall wird hierbei Druckluft verwendet. Von diesem Ventil 9 führt eine Leitung 11 zu einer Mehrzahl um den Brustkorb und/oder teilweise Bauch des Patienten 6 herum angeordneten geschlossenen Kammern 12 aus flexiblem Material. Um die Kammern herum befindet sich ein der Form des Brustkorbes angepasster Hohlkörper 13 aus starrem Material. Anstelle von Druckgas kann zum Füllen der Kammern auch eine Flüssigkeit verwendet werden. An die Leitung 11 ist wiederum ein Auslassventil 14 angeschlossen.

Weiterhin enthält der Ventilator 1 einen Verstärker 15, dem die Signale eines Sensors zugeführt werden. Im vorliegenden Ausführungsbeispiel dienen dafür zwei EKG-Elektroden 36 und 37, die auf dem Körper des Patienten appliziert sind und die elektrischen Herzsignale registrieren. Ueber den Verstärker 15 gelangen diese Signale auf einen Detektor 16, der die hohe elektrische Herzspannung während der Systole detektiert und der einen Impuls an elektronische Kreise 17 bzw. 18 abgibt. Diese Kreise 17 bzw. 18 sollen Mittel zum Einstellen einer wählbaren Verzögerung des vom Detektor 16 kommenden Impulses enthalten. Ueber Impulsformungskreise 19, 20 bzw. 21 werden die Anordnung 11 sowie die Ventile 8, 9 und 14 angesteuert. Die Impulsformungskreise 19 bis 21 können beispielsweise monostabile Kippstufen mit unterschiedlicher Impulsbreite sein. Weiterhin sollen diese Impulsformungskreise Mittel zum Einstellen der Impulsbreite enthalten.

Weiterhin sind in der schematischen Darstellung gemäss des Figur die Lungen 25 und 26 des Patienten dargestellt, die grosse Teile des Herzens umschliessen, von dem die linke Kammer 27 dargestellt ist.

Durch die Ausdehnung der Lungen steigt der Druck auf das Herz. Diese Drucksteigerung ist unter normalen Umständen jedoch sehr gering, da sich das Volumen des Brustkorbes teilweise durch auswärts gerichtete Bewegung der Brustkorbswände 28 und teils durch abwärts gerichtete Bewegung des Zwerchfelles in die Bauchhöhle leicht vergrössert.

Nach einer Ausgestaltung der Erfindung ist der Brustkorb und der obere Teil des Bauches von einem Hohlkörper 13 umschlossen, der aus einer formbaren Hülle aus festem Gewebe besteht. Diese Hülle wird so um den Körper herum angebracht, dass eine gewisse Ausdehnung der Atmungsorgane, d.h. der Lungen, des Brustkorbes und des Bauches möglich ist, ohne dass die Hülle diese Ausdehnung wesentlich behindert. Wenn diese Ausdehnung durch Zufuhr von Atemgas eine gewisse Grösse erreicht hat, wird die weitere Ausdehnung durch die Hülle verhindert, wodurch ein gesteigerter Druck innerhalb dieser Hülle und damit auch innerhalb des Brustkorbes und um das Herz herum entsteht.

Die Funktion des Respirators und damit auch das erfindungsgemässe Verfahren wird im folgenden näher beschrieben. Die elektrische Aktivität des Herzens zu Beginn einer Systole wird durch die Elektroden 16 und 17 registriert und löst die Zufuhr von Atemgas über die Anordnung 3 aus. Die physiologische Verzögerung zwischen dem elektrischen Herzsignal für die Systole (QRS-Komplex) und der mechanichen Kontraktion des Herzens wird ausgenutzt, um die Lungen über die bekannte schnelle Anordnung 3 mit einem Atemzug zu füllen. Es ist aber ebenso möglich, in den elektrischen Kreisen 17 und 18 eine derartige Verzögerung einzustellen, dass die Zufuhr von Atemgas während einer Systole geschieht, die mit einem nachfolgenden Herzschlag zusammenhängt.

Gleichzeitig mit der Atemgaszufuhr oder nahezu gleichzeitig damit werden die elastischen Kammern 12 über das Ventil 9 und die Leitung 11 mit Gas gefüllt. Die zugeführte Gasmenge kann dabei wiederum geregelt werden. Wenn die Lungen sich aufgrund des zugeführten Atemgases ausdehnen, entsteht ein Ueberdruck, der in dem Augenblick verstärkt wird, wenn die auswärts bzw. abwärts gerichtete Bewegung der Brustkorbswand 28 oder des Zwerchfelles 29 durch den Hohlkörper 13 und teilweise durch die gefüllten Kammern 12 gestoppt wird. Dieser Ueberdruck pflanzt sich durch die Herzwände fort, so dass der Druck auf das im Herzen eingeschlossene Blut ansteigt. Durch entsprechende Einstellung der elektrischen Kreise 17, 18, 19 20 und 21 wird dafür gesorgt, dass die Drucksteigerung zeitlich mit dem Herauspumpen des Blutes aus der linken Herzkammer 27 in die grosse Körperpulsader 26 und von da weiter zu wichtigen Organen sowie dem Gehirn über dessen Arterien 31 erfolgt.

Die Einstellung der verschiedenen Verzögerungszeiten sowie Impulsdauern geschieht nach folgenden Prinzipien: Die Atemgasmenge, die bei jedem Atemzug dem Patienten zugeführt wird, wird durch Einstellen der Anordnung 3 und/oder des Druckes in der Leitung 2 bestimmt, so dass die Ventilation der Lungen für einen guten Gasaustausch ausreicht. Der Hohkörper 13 wird um den Patienten herum derart angebracht, dass er sich den äusseren Konturen des Körpers anpasst, ohne zu Beginn eines Atemzuges grossen Druck auf diesen auszuüben. Ebenso wird die Zufuhr von Luft zu den geschlossenen Kammern 12 so gesteuert, dass die Bewegungen des Brustkorbes und des Zwerchfelles während der Zufuhr von Atemgas in dem Masse begrenzt wird, dass eine passende Steigerung des Druckes um das Herz herum während des Pumpvorganges desselben erzeugt wird.

Der Brustkorb selbst wird durch die flexiblen Kammern 12 nicht in dem Masse komprimiert, dass die dadurch ausgeübte Kraft eine inwärts gerichtete Bewegung der Brustkorbswände 28 hervorruft. Die flexiblen Kammern 12 füllen lediglich die Hohlräume zwischen der Köperoberfläche und dem Hohlkörper 13 aus. Sie dienen dazu, die auswärts gerichtete Bewegung der Körperoberfläche definiert und steuerbar zu begrenzen. Ausserdem dienen sie dazu, gewisse Unebenheiten des Druckes auf die Körperoberfläche auszugleichen, die beispeilsweise ein steifer Hohlkörper hervorrufen könnte.

Der Druck in der Lungen, der bei der Atemgaszufuhr entsteht, setzt sich zusammen aus dem Druck, der erforderlich ist zum Ausdehnen der Lungen und der Thoraxwände und dem Druck der im Brustkorb aufgrund des Hohlkörpers und der flexiblen Kammern 12 entsteht. Der Druckgradient über der Lungenstruktur übersteigt dabei jedoch nicht den Gradienten, der bei einer gängigen hochfrequenten Beatmung auftritt und der erfahrungsgemäss unschädlich ist. Irgend eine deformierende Kraft, die die Thoraxwände beschädigen könnte, wird durch den Druckausgleich der luftgefüllten flexiblen Kammern 12 verhindert. Eine schädliche Auswirkung auf die Blutzirkulation durch ein behindertes Wiederauffüllen der herzkammer wird dadurch vermieden, dass jede Form von ungewünschtem Druck auf den Brustkorb und in diesem während der Entspannungsphase des Herzens verhindert wird, indem sich die flexiblen Kammern 12 am Ende der Systole über das Ventil 14 entleeren. Gleichzeitig entleeren sich die Lungen über das Ventil 8.

Die elektrische Kopplung gemäss dem Ausführungsbeispiel ist folgende:

Das Ausgangssignal des Detektors 16 gelangt über die beiden Verzögerungskreise 17 bzw. 18 auf die Impulsformungskreise 19 bzw. 20. Der Impulsformungskreis 19 bestimmt die Zeitdauer, während deren Atemgas über die Anordnung 3 dem Patienten zugeführt wird und auch den Zeitpunkt, zu dem diese Zufuhr beginnen soll. Entsprechend bestimmt der Kreis 20, wann und wie lang das Ventil 9 geöffnet sein soll. Ueber den weiteren Kreis 21 wird der Zeitpunkt für das Öffnen der Ventile 14 bzw. 8 festgelegt. Gleichzeitig ist hier vorausgesetzt, dass die Ventile 8 bzw. 14 geschlossen sind, wenn die Anordnung 3 bzw. das Ventil 9 öffnen.

**Patentansprüche**

1. Respirator (1) zur niederfrequenten Beatmung eines Patienten (6) im Herzrhythmus und zur Unterstützung der Blutzirkulation mit einer ersten ventilgesteuerten Anordnung (3) für die Atemgaszufuhr, gekennzeichnet durch einen Sensor (16, 17) zum Erfassen eines mit der Herzaktivität gekoppelten Parameters sowie einer Steuereinrichtung (15—21), die in Abhängigkeit von dem Sensorsignal die Ventile der ersten Anordnung (3) derart betätigt, dass das Atemgas zu einem Zeitpunkt im Herzzyklus zugeführt wird, dass die dadurch verursachte Drucksteigerung um das Herz herum mit der Kontraktionsphase des Herzens zusammenfällt, sowie eine weitere Anordnung (12, 13), die zumindest Teile des Brustkorbes und Bauches umschliesst und aus einer Reihe geschlossener Kammern (12) aus einem flexiblen Material besteht, die mit einer Flüssigeeit oder einem Gas derart füllbar sind, dass die Kammern (12) phasengerecht zur Atemgaszufuhr durch die erste Anordnung eine Kraft auf Brustkorb und Bauch ausüben, die der Ausdehnung von Brustkorb und Bauch entgegenwirkt.

2. Respirator nach Anspruch 1, dadurch gekennzeichnet, dass der Zeitpunkt und/oder die Dauer und/oder die Grösse der Füllung der Kammern (12) einstellbar sind.

3. Respirator nach Anspruch 2, dadurch gekennzeichnet, dass die Steuereinrichtung einstellbare Verzögerungsglieder (17—21) aufweist, mit deren Hilfe der Zeitpunkt für die Atemgaszufuhr und/oder deren Dauer und/oder der Zeitpunkt für das Füllen bzw. Entleeren der Kammern (12) im Herzzyklus festlegbar ist.

4. Respirator nach einem der vorhergehenden Ansprüche, dadurch gekennzeichnet, dass als Sensor EKG-Elektroden (36, 37) vorgesehen sind, die auf die Haut des Patienten (6) auflegbar sind.

**Revendications**

1. Respirateur (1) pour réaliser la ventilation à basse fréquence d'un patient (6) en synchronisme avec son rythme cardiaque et assister la circulation sanguine, comportant un premier dispositif (3) commandé par une soupape et servant à introduire le gaz respiratoire, caractérisé par un capteur (16, 17) servant à détecter un paramètre couplé à l'activité cardiaque, ainsi qu'un dispositif de commande (15—21) qui, en fonction du signal du capteur, actionne les soupapes du premier dispositif (3) en sorte que le gaz respiratoire est délivré à un instant du cycle cardiaque, que l'accroissement de pression ainsi provoqué autour du coeur coïncide avec la phase de contraction de ce dernier, et un autre dispositif (12, 13), qui en toure au moins des parties de la cage thoracique et de l'abdomen et est constitué par une série de chambres fermées (12) réalisées en un matériau flexible et pouvant être remplies par un liquide ou un gaz de sorte que les chambres (12) exercent sur la cage thoracique et l'abdomen, et ce selon une phase correcte pour l'envoi du gaz respiratoire à travers le premier dispositif, une force qui s'oppose à la dilatation de la cage thoracique et de l'abdomen.

2. Respirateur suivant la revendication 1, caractérisé par le fait que l'instant et/ou la durée et/ou le volume de remplissage des chambres (12) sont réglables.

3. Respirateur suivant la revendication 2, caractérisé par le fait que le dispositif de commande comporte des circuits de retard réglables (17—21), à l'aide desquels l'instant d'envoi du gaz respiratoire et/ou sa durée et/ou l'instant de remplissage ou du vidage des chambres (12) pendant le cycle cardiaque peuvent être fixés.

4. Respirateur suivant l'une des revendications précédentes, caractérisé par le fait qu'il est prévu, comme capteur, des électrodes (36, 37) pour l'enregistrement d'électrocardiogrammes, qui peuvent être appliquées sur la peau du patient (6)

**Claims**

1. A respirator (1) for the low-frequency respiration of a patient (6) in cardiac rhythm and for assisting blood circulation, with a first valve-controlled arrangement (3) for the respiratory gas supply, characterized by a sensor (16, 17) for registration of a parameter associated with the heart activity, as well as a control device (15—21), which activates the valve of the first arrangement (3) depending on the sensor signal in such a way that the respiratory gas is supplied at a moment in the cardiac cycle such that the pressure increase around the heart caused thereby coincides with the contraction phase of the heart, and a further arrangement (12, 13), which surrounds at least parts of the thorax and abdomen and consists of a series of closed chambers (12) of a flexible material, which can be filled with a liquid or a gas in such a way that the chambers (12) exert a force on the thorax and abdomen appropriately phased with the respiratory gas supply by the first arrangement, which force counteracts the expansion of thorax and abdomen.

2. A respirator according to Claim 1, characterized in that the moment and/or the duration and/or the magnitude of the filling of the chambers (12) are adjustable.

3. A respirator according to Claim 2, characterized in that the control device has adjustable delay elements (17—21), with the aid of which the moment for the respiratory gas supply and/or its duration and/or the moment for the filling or evacuation of the chambers (12) can be fixed in the cardiac cycle.

4. A respirator according to one of the preceding claims, characterized in that ECG electrodes (36, 37) which can be laid on the skin of the patient (6), are provided as sensors.